# EUROPÄISCHE PATENTSCHRIFT

(11) **EP 3 833 312 B1**
(45) Veröffentlichungstag und Bekanntmachung des Hinweises auf die Patenterteilung: **07.05.2025**
(21) Anmeldenummer: 20702418.3
(22) Anmeldetag: 22.01.2020
(51) Int. Cl.: A61F 13/15, A61F 13/49, B32B 3/28, B32B 5/02, B32B 7/04, B32B 25/10, B32B 27/12, B32B 37/15

(54) **DEHNBARES WINDELELEMENT**
ELASTIC DIAPER ELEMENT
ÉLÉMENT ÉOLIEN ÉLASTIQUE

(30) Priorität: 20.02.2019 DE 102019104225
(43) Veröffentlichungstag der Anmeldung: 16.06.2021
(73) Patentinhaber: RKW SE, 68309 Mannheim (DE)
(72) Erfinder: WILLING, Christoph, 48691 Vreden (DE); WALLER, Paul, 83026 Rosenheim (DE); SCHERER, Micheal, 83126 Flintsbach im Inn (DE); EPPING, Reinhard, 48599 Gronau-Epe (DE)
(74) Vertreter: Busch, Tobias
(86) Internationale Anmeldenummer: PCT/EP2020/051479
(87) Internationale Veröffentlichungsnummer: WO 2020/169298

(56) Entgegenhaltungen:
- EP-A1- 0 182 942
- EP-A1- 1 740 364
- EP-A1- 1 761 229
- EP-B1- 1 807 035
- WO-A1-03/075735
- WO-A1-95/34264

## Beschreibung

Die Erfindung betrifft ein dehnbares Windelelement mit einer elastischen Lage und mindestens einer Lage aus Nonwoven, wobei das Windelelement Verbindungsbereiche zwischen der elastischen Lage und der Lage aus Nonwoven aufweist. Die Nonwoven-Lage umfasst im ungedehnten Zustand des Windelelements gewellt ausbildete Bereiche als Reserve zur Ermöglichung einer Dehnung.

Bei Windeln kommen elastische Elemente zum Einsatz, um eine gute Passform und Dichtigkeit zu gewährleisten. Das erfindungsgemäße Windelelement kommt vorzugsweise als Windelbund, als sogenanntes "Waistband", zum Einsatz. Für die Verwendung als elastisches Verschlusselement, sog. "Back Ear" an Babywindeln, ist das Material ebenfalls besonders geeignet.

Das erfindungsgemäße Windelelement umfasst eine Lage aus einem elastischen Material. Die elastische Lage ist an mindestens einer Seite mit einer Lage aus einem Vliesstoff versehen. Solche Vliesstoffe weisen in der Regel eine begrenzte Dehnbarkeit auf. Bei den erfindungsgemäßen Windelbund-Elementen ist eine daher Nonwoven-Lage in einer gewellt ausgebildeten Form mit der elastischen Lage verbunden. Die Wellentäler sind mit der elastischen Lage verbunden, während die Wellenberge von der elastischen Lage wegragen und einen Hohlraum zur elastischen Lage hin einschließen. Durch die Wellen bildet die Nonwoven-Lage im ungedehnten Zustand eine Reserve zur Ermöglichung einer Dehnung des Elements.

Herkömmlicherweise werden solche Windelelemente dadurch hergestellt, dass zunächst die elastische Lage gedehnt wird und dann die Nonwovenlage flach auf die elastische Lage aufgebracht wird. Die Nonwoven-Lage wird dann mittels Ultraschallverschweißung bereichsweise mit der elastischen Lage verbunden. Nach Entspannung des Laminats bildet die Nonwoven-Lage die gewünschte gewellte Struktur aus.

In der DE 689 23 866 T2 wird eine Windel mit einer oberen und einer unteren Schicht beschrieben. Es erfolgt eine Befestigung eines elastischen Bandes an der elastischen Schicht im ungespannten Zustand. Das elastische Band wird ganzflächig mit der elastischen Schicht verbunden. Nach dem Dehnen und Entspannen des elastischen Bandes bilden sich geraffte Bereiche aus.

Die EP 217032 B1 betrifft ein Laminat mit einem elastischen Material, das an zueinander beabstandeten Stellen mit mindestens einer in Falten zu legenden Bahn verbunden ist. Bei dem elastischen Material handelt es sich um eine nicht gewellte, elastische Faserbahn.

In der EP 1 807 035 B1 wird ein Verfahren zur Herstellung eines gewellten Dehnungslaminates beschrieben. Dabei wird eine elastische Zusammensetzung in einem geschmolzenen Zustand auf eine Trägerbahn zur Bildung eines elastischen Elements aufgebracht. Es erfolgt die Bildung einer Dehnungsverbund-Vorform durch Dehnen der Trägerbahn. Dann erfolgt ein Strecken der Vorform. Ein Substrat wird mit der gestreckten Vorform verbunden, um ein gewelltes Dehnungslaminat bei einer Entspannung der gestreckten Vorform zu bilden.

Die EP 2 024 178 B1 beschreibt ein Verfahren zur Herstellung eines elastisch dehnbaren Laminats mit drei Lagen. Das Laminat umfasst eine elastische Folie und zwei Lagen aus nicht elastischem Vliesgewebe. Bei einer Variante kommt ein Kreppvlies zum Einsatz. Ein erstes elastisches Laminat wird mit einer nicht elastischen Vlieslage in einem gedehnten Zustand verbunden.

Aufgabe der vorliegenden Erfindung ist es, ein Windelelement bereitzustellen, das günstige Dehneigenschaften aufweist und gleichzeitig eine hohe Reißfestigkeit gewährleistet. Das Element soll sich bei einer Kraftbeanspruchung dehnbar verhalten, aber auch einen ausreichenden Widerstand aufbauen, der dem Verbraucher ein Gefühl eines hochwertigen Produkts verleiht. Bei der Verwendung als "Back Ear" darf das Element nicht reißen da sich ansonsten die Windel nicht mehr verschließen lässt. Dabei soll mindestens eine Nonwoven-Lage durch eine wellige Ausbildung eine ausreichende, aber nicht zu große Reserve für eine Streckung des Elements bereitstellen. Das Laminat soll gesundheitlich unbedenklich und ökologisch nachhaltig sein. Zudem sollen keine Gerüche von dem Produkt ausgehen. Weiterhin soll das Element eine angenehme Haptik aufweisen. Zudem soll durch das Windelelement eine optimale Passform der Windel am Körper gewährleistet werden, so dass Hohlräume zwischen Windel und Körper vermieden werden und ein Auslaufen von Flüssigkeit verhindert wird.

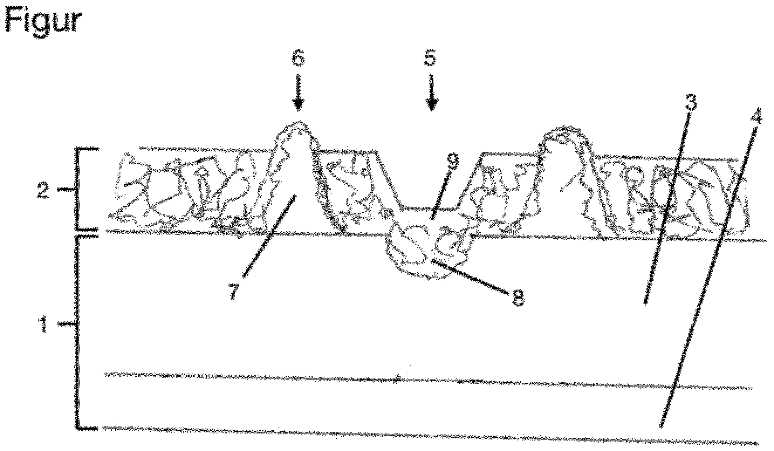

Diese Aufgabe wird erfindungsgemäß durch ein Windelelement gemäß Anspruch 1 gewährleistet. Bevorzugte Varianten sind den Unteransprüchen, der Beschreibung, dem Ausführungsbeispiel und der Zeichnung zu entnehmen.

Gemäß der Erfindung umfassen die Verbindungsbereiche Zonen, bei denen ein formschlüssiger Verbund von Material der Lage aus Nonwoven und erstarrtem Material der elastische Lage vorliegt, wobei in den Verbindungsbereichen Fasern der Lage aus Nonwoven orientiert und/oder gedehnt vorliegen, wobei die elastische Lage in Form einer elastischen Folie ein spezifisches Flächengewicht von 10 bis 130 g/m² aufweist.

Erfindungsgemäß sind die Verbindungsbereiche zwischen der gewellten Nonwoven-Lage und der elastischen Lage durch einen formschlüssigen Verbund ausgebildet, bei dem Nonwoven-Material im erstarrten Material der elastischen Schicht vorliegt. Im Gegensatz zu herkömmlichen Waistband-Laminaten wird zur Herstellung der erfindungsgemäßen Laminate zunächst durch eine Vorrichtung aus einer glatten Nonwoven-Lage eine gewellte Struktur durch Verformung der Oberfläche gebildet. Dadurch bilden sich Vertiefungen und Erhebungen aus, also Wellen und Täler. Dann wird eine elastische Lage zu der gewellt ausgebildeten Nonwoven-Lage extrudiert. Die Vertiefungen der Nonwoven-Lage werden gezielt in die schmelzflüssige elastische Lage gedrückt. Nach einer Erstarrung der elastischen Lage bilden sich formschlüssige Verbindungsbereiche aus, bei denen Nonwoven Material in dem erstarrten elastischen Material vorliegt. Die Erhebungen der Nonwoven-Lage ragen von der elastischen Lage weg und bilden eine Dehnungsreserve. Im Bereich der Dehnungsreserve sind Hohlräume zwischen der Nonwoven-Lage und der elastischen Lage eingeschlossen.

Im Gegensatz zu herkömmlichen Verfahren liegt die elastische Lage bei der Bildung des Laminats nicht als Folie, sondern in schmelzflüssiger Form vor und wird nicht vor dem Verbindungsschritt gedehnt. Weiterhin wird die Nonwoven-Lage beim Verbindungsschritt nicht flach, sondern in gewellter Form auf die elastische Lage aufgebracht.

Bei dem Verbindungsvorgang erfolgt keine Wärmeeinwirkung von außen, wie dies beispielsweise bei Anwendung einer Ultraschallverschweißung der Fall wäre. Bei dem erfindungsgemäßen Laminat erfährt das gewellte Nonwoven-Material somit keine thermische Belastung von außen. Dadurch wird beispielsweise ein Pinholing der Folie vermieden, wie es bei herkömmlichen Verbindungsverfahren, z.B. beim Ultraschallverschweißen, auftreten kann. Zur Herstellung des Laminats sind keine Kleber erforderlich. Es erfolgt keine Verpressung über die Gesamtfläche.

Im Folgenden wird die Erfindung detailliert beschrieben.

Analog zum dem Ausdruck "gewellt" können auch die Ausdrücke "geriffelt", "gefältelt", "gerafft", "gekräuselt", "gerippt", "gefaltet" oder "gekreppt", verwendet werden und beschreiben die räumliche Ausrichtung des Vliesstoffes. Erfindungsgemäß wird die wellenförmige Ausgestaltung der Nonwoven-Lage dadurch erzielt, dass der Vliesstoff vor dem Verbindungsprozess über eine spezielle Vorrichtung geführt wird, beispielsweise eine geripptes Führungselement, eine Fingerleiste, eine Rippenwalze oder eine Kammwalze.

Bei einer Variante der Erfindung kann das gewellte Profil der Nonwoven-Lage zwischen zwei Walzen gebildet werden, die Erhebungen und Vertiefungen aufweisen, wobei mindestens eine der beiden Walzen als Kammwalze ausgebildet ist. Die Erhebungen der einen Walze greifen dabei in die Vertiefungen der anderen Walze ein und umgekehrt.

Bei einer alternativen Variante der Erfindung wird das gewellte Profil der Nonwoven-Lage zwischen einer Walze und einem weiteren Element gebildet, das wie die Walze auch Erhebungen und Vertiefungen aufweist. Die Erhebungen der Walze greifen dabei in die Vertiefungen des Elements ein und umgekehrt. Das Element erstreckt sich bogenförmig bis zur extrudierten elastischen Lage, so dass das gewellte Profil der Nonwoven-Lage bis zu dem Verbindungsschritt erhalten bleibt.

Die Ausdrücke "Nonwoven" bzw. "Vlies" beziehen sich auf einen Stoff, der aus kontinuierlichen Filamenten und/oder diskontinuierlichen Fasern ohne Weben oder Stricken mittels Verfahren wie Spunbonding, Kardieren oder Meltblowing hergestellt werden kann. Der Vliesstoff kann eine oder mehrere Lagen Vlies umfassen, wobei jede Lage kontinuierliche Filamente oder diskontinuierliche Fasern enthalten kann. Vlies kann auch Bikomponentenfasern umfassen, welche Faserstrukturen wie zum Beispiel Mantel/Kern-, Seit-an-Seit aufweisen können.

Der Ausdruck "elastisch" bezieht sich vorzugsweise auf jedes Material, das sich nach Anlegen einer gerichteten Kraft auf eine gedehnte Länge von mindestens ungefähr 160% seiner entspannten, ursprünglichen Länge strecken kann, ohne zu reißen oder zu brechen und das sich nach dem Absetzen der angelegten Kraft um mindestens ungefähr 55 % seiner Verlängerung rückstellt, vorzugsweise im Wesentlichen auf seine ursprüngliche Länge, d.h. die rückgestellte Länge beträgt weniger als ungefähr 120%, vorzugsweise weniger als ungefähr 110%, mehr bevorzugt weniger als ungefähr 105% der entspannten ursprünglichen Länge.

Der Ausdruck "Windel" bezieht sich vorzugweise auf Einweg-Absorptionsartikel, welche Fluide absorbieren und einschließen. Der Begriff umfasst unter anderem Windeln mit Verschlüssen, Windelhöschen, Übungshöschen, Schwimmwideln, Inkontinenzartikel für Erwachsene und dergleichen.

Bei der Herstellung des Windelelements wird die Nonwovenlage vor dem Verbindungsschritt in eine dreidimensionale, gewellte Form gebracht, indem sie über eine spezielle Vorrichtung geführt wird. Bei dieser Vorrichtung kann es sich um eine Walze handeln, die Erhebungen aufweist und dadurch das Wellenprofil der Nonwovenlage ausbildet. Ergänzend oder alternativ kann die Nonwovenlage vor dem Verbindungsschritt über ein Element geführt werden, das sich bogenförmig bis zur extrudierten elastischen Lage erstreckt, so dass das gewellte Profil der Nonwovenlage bis zu dem Verbindungsschritt erhalten bleibt. Das Element kann beispielsweise als Fingerleiste ausgebildet sein.

Die Herstellung des Laminats erfordert neben der Aufschmelzleistung des Extruders keine Energie von außen, wie dies beispielsweise beim Ultraschallverschweißen der Fall wäre. Die Schmelze erstarrt und es bilden sich die erfindungsgemäßen Verbindungsbereiche. Die Verbindungsbereiche werden somit auf eine Weise geschaffen, dass die Nonwoven-Lage keine thermische Belastung von außen erfährt, wie dies zum Beispiel beim Ultraschallverschweißen oder einem Aufschmelzen der elastischen Lage mittels Heizwalzen der Fall wäre.

Durch die selektive Erwärmung der elastischen Lage mittels Cast-Extrusion bei gleichzeitiger Erzeugung eines Wärmstroms von innen nach außen beim Verbindungsschritt bleibt die tragende Struktur der elastischen Schicht erhalten. Durch Gewährleistung eines Wärmestrom von innen nach außen beim Verbindungsvorgang wird ein Laminat geschaffen, das besonders günstige Eigenschaften als Windelbund aufweist.

Der Wärmestrom von innen nach außen kann gezielt gesteuert werden. Bei einer besonders günstigen Ausführung der Erfindung kommt dazu mindestens eine Kühlwalze zum Einsatz. Durch eine Absenkung der Oberflächentemperatur der Kühlwalze kann der Strom, der von innen nach außen strömenden Wärmeenergie erhöht werden.

Die elastische Lage liegt nach der Extrusion vorzugsweise oberhalb einer Temperatur von 180°C schmelzflüssig vor. Vorzugsweise erfolgt eine Abkühlung beim Verbindungsschritt um mehr als 80°C, vorzugsweise um mehr als 130°C.

Die erfindungsgemäßen Verbindungsbereiche sind vorzugsweise streifenartig in nebeneinander angeordneten Reihen ausgebildet. Innerhalb einer Reihe liegt vorzugsweise ein gerader Verlauf vor. Die Ausrichtung der Verbindungsbereiche ist vorzugsweise senkrecht zur Zugrichtung des Windelelements, so dass die einzelnen Reihen quer zur Zugrichtung des Windelbundes ausgerichtet sind.

Wichtig für ein optimales Windelbundlaminat ist das Verhältnis der hervorgehobenen zu den abgesenkten Bereichen der Vorrichtungen, die zur Bildung des Wellenprofils und beim Verbindungsschritt eingesetzt werden. Dieses Verhältnis wird auch als Steg-Nut-Verhältnis bezeichnet. Vorzugsweise beträgt dieses Steg zu Nut-Verhältnis weniger als 1:1, vorzugsweise weniger als 1:2.

Als besonders günstig erweist es sich, wenn die Verbindungsbereiche eine Breite von mehr als 0,1 mm, vorzugsweise mehr als 0,3 mm, insbesondere von mehr als 0,5 mm und/oder eine Breite von weniger als 1,5 mm, vorzugsweise weniger als 1,3 mm, insbesondere weniger als 0,9 mm aufweisen.

Bei einer bevorzugten Variante der Erfindung weisen die Verbindungsbereiche außenliegende Zonen auf, in denen kein elastisches Material eingedrungen ist. In den außenliegenden Zonen der Verbindungsbereiche liegt kein formschlüssiger Verbund vor, da diese Zonen frei sind von elastischem Material. Durch die außenliegenden Zonen wird ein Durchschlagen von elastischem Material durch die Nonwoven-Lage verhindert.

In den inneren Zonen der Verbindungsbereiche liegt ein formschlüssiger Verbund von erstarrtem elastischem Material und Nonwovenmaterial vor. Dabei ist das Nonwovenmaterial auch ein den inneren Zonen vorzugsweise nicht angeschmolzen, sondern die Fasern sind lediglich in die elastische Schmelze hineingedrückt. Das Material der elastischen Lage umgibt die Filamente der Nonwoven-Lage, so dass nach der Erstarrung der elastischen Lage ein formschlüssiger Verbund in der inneren Zone der Verbindungsbereiche entsteht.

Es kann in Einzelfällen auch vorkommen, dass in der inneren Zone zumindest einzelne Faser des Nonwovenmaterials angeschmolzen sein. Auch ist rein theoretisch möglich, dass in der inneren Zone das Nonwovenmaterial vollständig geschmolzen in der elastischen Schmelze vorliegt.

In allen Fällen liegt nach einer Erstarrung des elastischen Materials in der inneren Zone ein formschlüssiger Verbund von Nonwovenmaterial und erstarrtem Material der elastischen Schicht vor.

Zwischen den Verbindungsbereichen sind Bereiche angeordnet, in denen die gewellte Nonwovenlage mit ihren Erhebungen von der elastischen Lage wellenförmig wegragt und Hohlräume einschließt. Diese Bereiche dienen als Reserve für die Dehnung des Windelelements, wobei in diesen Bereichen keine Verbindung zwischen der Nonwoven-Lage und der elastischen Lage vorliegt.

Auf die Gesamtfläche der flachen Folie bezogen weisen die Reservebereiche einen deutlich größeren Anteil als die Verbindungsbereiche auf. Vorzugsweise beträgt der Anteil der Reservebereiche mehr als 60 %, insbesondere mehr als 70 %, bevorzugt mehr als 80 % der Gesamtfläche. Als Gesamtfläche wird die Oberfläche der erstarrten flachen elastischen Lage als Bezug herangezogen.

In den Reservebereichen liegt keine Verbindung der Nonwoven-Lage mit der elastischen Lage vor.

Durch die wellige Gestaltung ist die Nonwoven-Lage eines Laminatabschnitts deutlich länger als die elastische Lage. Vorzugsweise ist die Nonwoven-Lage um mehr als den Faktor 1,5, insbesondere um mehr als den Faktor 2,0, bevorzugt um mehr als den Faktor 2,5 länger als die elastische Lage.

Bei einer bevorzugten Variante der Erfindung erfolgt nach dem Verbindungsvorgang eine Streckung des Laminats in Querrichtung. Vorzugsweise erfolgt die Dehnung unterhalb der Bruchdehnung der Nonwoven-Lage.

Als besonders günstig erweist es sich, wenn die Nichtbindungsbereiche, also die Reservebereiche, eine Breite von mehr als 1,5 mm, vorzugsweise mehr als 2 mm, insbesondere mehr als 2,5 mm und/oder eine Breite von weniger als 6 mm, vorzugsweise weniger als 5 mm, insbesondere weniger als 4 mm aufweisen.

Die Bindungsbereiche weisen vorzugsweise eine Breite von mehr 0,1 mm, insbesondere mehr als 0,2 mm, bevorzugt mehr als 0,3 mm und/oder eine Breite von weniger als 1 mm, vorzugsweise weniger als 0,8 mm, bevorzugt weniger als 0,6 mm auf.

Die Reservebereiche bzw. Verbindungsbereiche sind vorzugsweise streifenförmig quer zur Zugrichtung des Windelbunds ausgebildet.

Zur Schaffung der Verbindungsbereiche kommen vorzugsweise Walzen mit einer Oberflächenstruktur zum Einsatz, wobei die Höhe der Erhebungen mehr als 100 µm, vorzugsweise mehr als 500 µm, insbesondere mehr als 1 mm und/oder weniger als 8 mm, vorzugsweise weniger als 10 mm, insbesondere weniger als 12 mm beträgt.

Vorzugsweise besteht die Nonwoven-Schicht aus einem wasserstrahl-verfestigten Vliesstoff. Durch eine Wasserstrahlvernadelung können Fasern im Vliesstoff umorientiert werden, sodass die ursprüngliche zweidimensionale Faserausrichtung in ein dreidimensionale Faserorientierung überführt wird. Die Fasern sind stärker in das Vlies eingebunden. Diese Nonwoven-Schicht weist vorzugsweise ein spezifisches Gewicht von 5 bis 80 g/m², vorzugsweise von 10 bis 70 g/m², insbesondere von 15 bis 35 g/m² auf.

Vorzugsweise handelt es sich bei der wasserstrahlverfestigten Vliesstofflage um Vliesstoffe aus Endlosfilamenten. Diese bieten aufgrund ihres Herstellungsprozesses einen Faserflor, der vorzugsweise schlaufenartig ausgebildet ist.

Als Material zur Herstellung der Endlosfilamente können spinnbare Polymere, wie beispielsweise Polyester, PLA, Polyolefine, insbesondere Polypropylen und Polyethylen zum Einsatz kommen.

Der erfindungsgemäße Einsatz von wasserstrahl-verfestigten Vliesstoffs als gewellte Nonwoven-Lage, die Reservebereiche für eine Dehnung des Windelelements bildet, ist besonders vorteilhaft. Durch die Wellenbildung verformt sich der wasserstrahl-verfestigte Vliesstoff derart, dass die Fasern in den Verbindungsbereichen gestreckt werden und dadurch vorzugsweise eine Orientierung erfahren. Dadurch werden besonders vorteilhafte Verbindungszonen geschaffen, bei denen das schmelzflüssige Material die gedehnten und ausgerichteten Filamente des wasserstrahl-verfestigten Vliesstoffs umschließt und nach der Erstarrung ein besonders günstiger formschlüssiger Verbund entsteht. Erstaunlicherweise wurde festgestellt, dass beim Einsatz eines wasserstrahl-verfestigten Vliesstoffs ein Windelelement mit besonders günstigen Eigenschaften geschaffen wird. Die Nonwoven-Lage aus dem wasserstrahl-verfestigten Vliesstoffs lässt sich besonders gut verformen. Die zugeführte Nonwovenbahn verliert beim Herstellungsprozess trotz der Wellenbildung so gut wie keine Breite.

Bei der elastischen Lage handelt es sich vorzugsweise um ein Polypropylen- - und/oder einem Polyethylen-Block-Copolymer. Die elastische Folie weist ein spezifisches Gewicht von 10 bis 130 g/m², bevorzugt von 20 bis 40 g/m² auf.

Alternativ kann die elastische Lage auch aus einem SBC (Styrol-Block-Copolymer) oder einem elastischen Polyurethan bestehen.

Bei einer Variante der Erfindung ist die elastische Lage mehrschichtig aufgebaut und vorzugsweise als Coexfolie ausgeführt. Diese umfasst bei einer vorteilhaften Variante eine Kernschicht, "Core-Layer" und eine im Vergleich dazu deutlich dünnere "Skin-Layer". Die Skin-Layer weist vorzugsweise ein spezifisches Gewicht weniger als 5 g/m², insbesondere weniger als 4 g/m², bevorzugt weniger als von 3 g/m² auf und/oder mehr als 0,3 g/m², insbesondere mehr als 0,6 g/m², bevorzugt mehr als von 0,9 g/m².

Bei einer Variante ist die Kernschicht zwischen zwei Skin-Layer-Außenschichten eingebettet.

Die Kernschicht besteht vorzugsweise aus einem elastischen Polyolefin oder einem SBC (Styrol-Block-Colymer) oder einem Polyurethan.

Die Skin-Layer besteht vorzugsweise aus einem Polyethylen, einem Polypropylen oder einem EVA (Ethylen-Vinylacetat-Copolymer).

Bei einer günstigen Variante der Erfindung werden gefüllte Polyolefine als Skin-Layer eingesetzt. Als Füllstoffe kommen beispielsweise mineralische Materialien wie Calciumcarbonat oder Talkum zum Einsatz. Der Füllstoffanteil beträgt vorzugsweise mehr als 60 Gew.-%, insbesondere mehr als 70 Gew.-%, bevorzugt mehr als 80 Gew.-%.

Zur Bildung der Skin-Layer können auch Blends zum Einsatz kommen. Dabei eignen sich beispielsweise Blends von Polyolefinen mit Polystyrol und/oder Blends von Polyolefinen mit PLA. Bei solchen Blends ist kein Füllstoff erforderlich.

Die Skin-Lage ist so gestaltet, dass sie eine Entblockung von der klebrigen Kernschicht gewährleistet. Zudem ist die Skin-Lage leicht verformbar und lässt sich gut Dehnen.

Bei einer Variante der Erfindung umfasst das Windelelement eine Nonwoven-Schicht aus einem kardierten Vliesstoff. Der eingesetzte kardierte Vliesstoff besteht vorzugsweise aus Polypropylenfasern und/oder aus Mischungen verschiedener Fasertypen, wie zum Beispiel aus Polypropylen/Viskose, Polypropylen/Polyamid, Polypropylen/Polyester usw. Der kardierte Vliesstoff kann auch aus Polypropylen und/oder Polyethylen Copolymer bestehen. Vorzugsweise beträgt das spezifische Flächengewicht des kardierten Vliesstoffs 10 bis 40 g/m², insbesondere zwischen 15 bis 25 g/m². Der kardierte Vliesstoff kann beispielsweise mittels eines Kalanders und/oder mittels Lufteinwirkung und/oder Wasserstrahl verfestigt sein.

Bei dem Windelelement kann die elastische Lage auch zwischen zwei Lagen aus Nonwoven eingebettet sein. Dabei besteht vorzugsweise mindestens eine Lage aus einem wasserstrahl-verfestigten Vliesmaterial. Die zweite Nonwovenlage kann entweder auch aus einem wasserstrahl-verfestigten Vliesstoff oder einem kardierten Vliesstoff oder einem Spinnvliesstoff bestehen. Die zweite Nonwoven-Lage kann entweder auch gewellt ausgebildet sein oder ein flaches Profil aufweisen.

Durch die erfindungsgemäße Gestaltung des Windelelements liegt die Windel optimal am Körper an und gewährleistet eine optimale Passform. Durch die Höhe seines Faltenwurfs weist das Windelelement eine voluminöse Gestaltung auf und füllt Hohlräume zwischen Windel und Körper aus, so dass eine Leckage wirksam verhindert wird.

Weitere Vorteile und Merkmale der Erfindung ergeben sich aus der Beschreibung eines Ausführungsbeispiels anhand einer Zeichnung und aus der Zeichnung selbst.

Die Figur zeigt einen Schnitt durch ein erfindungsgemäßes Windelelement. Das Laminat umfasst eine elastische Lage 1 und eine Nonwoven-Lage 2 aus einem gewellten Vliesstoff. Dabei handelt es sich um einen wasserstrahlverfestigten Vliesstoff, wobei im Ausführungsbeispiel ein Spinnvlies aus Endlosfilamenten zum Einsatz kommt. Erfindungsgemäß wird der Vliesstoff vor der Verbindung mit der elastische Lage 1 in eine Wellenform gebracht. Nach einem Extrudieren der elastischen Lage 1 zu der gewellten Nonwoven-Lage 2 werden die Vertiefungen des Vliesstoffes in die schmelzflüssige elastische Lage 1 gedrückt, so dass sich Verbindungsbereiche 5 zwischen dem gewellten Vliesstoff 2 und der elastischen Lage 1 ausbilden.

Im Ausführungsbeispiel ist die elastische Lage 1 als mehrschichtige Coexfolie aufgeführt, mit einer Kernschicht 3 und einer weiteren Schicht 4, die als "Skin-Layer" ausgebildet ist. Das Verhältnis der Dicke der Kernschicht 3 zur weiteren Schicht 4 beträgt vorzugsweise mehr als 8:1 insbesondere mehr als 10:1 insbesondere mehr als 12:1: Die Skin-Layer 4 weist vorzugsweise ein Gewicht zwischen 1 bis 3 g/m² auf.

Vorzugsweise besteht die Kernschicht 3 aus thermoplastischen Polymeren. Dabei kommen vorzugsweise Polypropylen-Polyethylen-Block-Copolymere zum Einsatz, beispielsweise der Typenreihe Exxon Vistamaxx (PP basiert): VM 6102, oder VM6202 oder VM 7810 und/oder der Typenreihe Dow Infuse (PE basiert): Infuse 9507, Infuse 9107.

Die Außenschicht 4 besteht vorzugsweise aus einem Polyolefin oder einem Ethylen-Vinylacetat-Copolymer (EVA). Die Außenschicht 4 ist im Gegensatz zur Kernschicht 3 nicht "klebrig" und verhindert somit ein unerwünschtes Anhaften.

Erfindungsgemäß umfasst das Laminat Verbindungsbereiche 5 und Reservebereiche 6. Die Reservebereiche 6 weisen keine oder nur eine sehr schwache Bindung mit der elastischen Lage 1 aus und schließen vorzugsweise Hohlräume 7 ein.

Im Ausführungsbeispiel beträgt das Steg zu Nut-Verhältnis der Walze, welche das Nonwoven in eine Wellenform bringt und die Vertiefungen der gewellten Nonwoven-Lage 2 in die schmelzflüssige elastische Lage 1 drückt bei 1:6, vorbei die Stegbreite vorzugsweise 0,5 mm und die Nutbreite vorzugsweise 3 mm beträgt.

Die erfindungsgemäßen Verbindungsbereiche 5 weisen im Ausführungsbeispiel unterschiedliche Zonen 8, 9 auf.

Die außenliegende Zone 9 ist frei von elastischem Material, so dass kein Durchschlagen des elastischen Materials durch die Nonwoven-Lage 2 erfolgt. Das Nonwovenmaterial in der außenliegenden Zone 8 von außen thermisch unbeeinflusst, so dass die Filamente der Lage 2 aus Nonwoven nicht angeschmolzen sind.

In der inneren Zone 8 der Verbindungsbereiche 5 liegt ein formschlüssiger Verbund von erstarrtem elastischem Material und Nonwovenmaterial vor. Dabei ist im Ausführungsbeispiel auch in der inneren Zone 8 das Nonwovenmaterial nicht angeschmolzen. Die Endlosfilamente des wasserstrahl-verfestigten Vliesstoffs sind lediglich in die elastische Schmelze hineingedrückt, so dass nach der Erstarrung ein formschlüssiger Verbund entsteht. Dabei bleiben beim Verbindungsprozess die Endlosfilamente des wasserstrahl-verfestigten Vliesstoffs selbst weitgehend unbeeinflusst. Sie werden lediglich von dem schmelzflüssigen Material der elastische Lage 1 umschlossen.

Nach einer Erstarrung des elastischen Materials liegt in der inneren Zone 8 ein formschlüssiger Verbund von Nonwovenmaterial und erstarrtem Material der elastischen Schicht 1 vor.

Das in der Figur dargestellte Laminat wird zwischen einem Walzenpaar miteinander verbunden, bei der mit Blick auf die Zeichnung von oben eine profilierte Walze mit Erhebungen die Nonwoven-Lage 2 in die elastische Lage 1 drückt und von unten eine Gegenwalze mit einer glatten Oberfläche angeordnet ist. Bei der Herstellung des in der Figur dargestellten Laminats kommt eine Kühlwalze als Gegenwalze zum Einsatz. Bei der Kühlwalze handelt es sich um eine Stahlwalze. Bei der von oben wirkenden Walze handelt es sich um eine nicht gekühlte Walze.

Die zur Verbindung eingesetzten Walzen werden auf Abstand gefahren, wobei ein fester Abstand eingestellt wird.

Das Laminat hat vorzugsweise ein spezifisches Flächengewicht von mehr als 10 g/m², insbesondere mehr als 20 g/m², bevorzugt mehr als 30 g/m² und/oder weniger als 200 g/m², insbesondere weniger als 150 g/m², bevorzugt weniger als 100 g/m².

Im Ausführungsbespiel sind die Verbindungsbereiche 5 und die Reservebereiche 6 streifenförmig ausgeführt, wobei die Streifen quer zur Zugrichtung des Windelelements verlaufen.

Die Streifen der Verbindungsbereiche 5 haben ein Breite zwischen 0,1 und 1 mm. Im Ausführungsbeispiel haben die Verbindungsbereiche 5 eine Breite von 0,5 mm.

Die Streifen der Reservebereiche 6, bei denen keine Verbindung der Nonwoven-Lage 2 mit der elastisch Lage 1 vorliegt, haben eine Breite zwischen 2 und 6 mm. Im Ausführungsbeispiel haben die Reservebereiche 6 eine Breite von 3 mm. Somit haben im Ausführungsbeispiel die Verbindungsbereiche 5 einen Anteil von 0,5/3,5 = 14,3 % und die Reservebereiche 6 einen Anteil von 3/3,5 = 85,7 % bezogen auf die Oberfläche der flachen, nicht gedehnten elastischen Lage 1.

## Patentansprüche

1. Dehnbares Windelelement mit einer elastischen Lage (1) und einer Lage (2) aus Nonwoven, wobei das elastische Element Verbindungsbereiche (5) zwischen der elastische Lage (1) und der Lage (2) aus Nonwoven aufweist, wobei die Lage (2) aus Nonwoven im ungedehnten Zustand des Windelelements gewellt vorliegt, um Bereiche (6) als Reserve zur Ermöglichung einer Dehnung zu gewährleisten, **dadurch gekennzeichnet, dass** die Verbindungsbereiche (5) Zonen (8) umfassen, bei denen ein formschlüssiger Verbund von Material der Lage (2) aus Nonwoven und erstarrtem Material der elastische Lage (1) vorliegt, wobei in den Verbindungsbereichen (5) Fasern der Lage (2) aus Nonwoven orientiert und/oder gedehnt vorliegen, wobei die elastische Lage (1) in Form einer elastischen Folie ein spezifisches Flächengewicht von 10 bis 130 g/m² aufweist.

2. Windelelement nach Anspruch 1, **dadurch gekennzeichnet, dass** die Verbindungsbereiche (5) Außenzonen (9) aufweisen, in denen kein Material der elastischen Lage (1) eingedrungen ist, so dass kein Durchschlagen der elastischen Lage (1) durch die Lage (2) aus Nonwoven erfolgt.

3. Windelelement nach Anspruch 2, **dadurch gekennzeichnet, dass** in den Außenzonen (9) das Nonwoven-Material nicht angeschmolzen ist.

4. Windelelement nach einem der Ansprüche 1 bis 3, **dadurch gekennzeichnet, dass** in den Zonen (8), in denen ein formschlüssiger Verbund von Nonwoven-Material und erstarrtem elastischen Material vorliegt, das Nonwoven-Material nicht angeschmolzen ist.

5. Windelelement nach einem der Ansprüche 1 bis 4, **dadurch gekennzeichnet, dass** die Lage (2) aus Nonwoven aus einem wasserstrahlverfestigten Vliesstoff besteht, vorzugsweise aus einem wasserstrahlverfestigten Vliesstoff aus Endlosfilamenten, wobei der Vliesstoff vorzugsweise ein spezifisches Gewicht von 10 bis 70 g/m², insbesondere 10 bis 40 g/m², bevorzugt 15 bis 35 g/m² aufweist.

6. Windelelement nach einem der Ansprüche 1 bis 5, **dadurch gekennzeichnet, dass** die elastische Lage (1) mehrschichtig aufgebaut ist und vorzugsweise eine Kernschicht (3) und mindestens eine weitere Schicht (4) aufweist.

7. Windelelement nach einem der Ansprüche 1 bis 6, **dadurch gekennzeichnet, dass** das Element eine weitere Lage aus Nonwoven aufweist, wobei die elastische Lage (1) zwischen den beiden Lagen aus Nonwoven angeordnet ist.

## Claims

1. Elastic diaper element comprising an elastic layer (1) and a layer (2) of non-woven, wherein the elastic element comprises connecting regions (5) between the elastic layer (1) and the layer (2) of non-woven (2) wherein the layer (2) of nonwoven is corrugated in the unstretched state of the diaper element, in order to ensure areas (6) as a reserve to enable stretching, **characterized in that** the connecting regions (5) comprise zones (8) in which a form-fit bond of material of the layer (2) made of nonwoven and solidified material of the elastic layer (1) is present, wherein fibers of the nonwoven layer (2) are present being oriented and/or stretched in the connecting regions (5), the elastic layer (1) in the form of an elastic film having a specific weight per area of 10 to 130 g/m².

2. Diaper element according to claim 1, **characterized in that** the connecting regions (5) have outer zones (9) in which no material of the elastic layer (1) has penetrated, so that there is no penetration of the elastic layer (1) by the nonwoven layer (2).

3. Diaper element according to claim 2, **characterized in that** in the outer zones (9), the nonwoven material is not melted.

4. Diaper element according to one of claims 1 to 3, **characterized in that** in the zones (8) in which a form-fit bond of nonwoven material and solidified elastic material exists, the nonwoven material is not melted.

5. Diaper element according to one of claims 1 to 4, **characterized in that** the layer (2) of nonwoven consists of a hydroentangled nonwoven fabric, preferably of a hydroentangled nonwoven fabric of continuous filaments, the nonwoven fabric preferably has a specific weight of 10 to 70 g/m², in particular 10 to 40 g/m², preferably 15 to 35 g/m².

6. Diaper element according to one of claims 1 to 5, **characterized in that** the elastic layer (1) has a multilayer structure and preferably comprises a core layer (3) and at least one further layer (4).

7. Diaper element according to one of claims 1 to 6, **characterized in that** the element has a further layer of nonwoven fabric, the elastic layer (1) being arranged between the two layers of nonwoven fabric.

## Revendications

1. Elément de couche extensible comprenant une couche élastique (1) et une couche (2) de non-tissé, l'élément élastique comportant des régions de liaison (5) entre la couche élastique (1) et la couche (2) de non-tissé, la couche (2) de non-tissé étant ondulée dans l'état non étiré de l'élément de couche pour fournir des zones (6) de réserve pour permettre l'étirement, **caractérisé en ce que** les régions de liaison (5) comprennent des zones (8) dans lesquelles une liaison par complémentarité de forme de matériau de la couche (2) de non-tissé et de matériau solidifié de la couche élastique (1) est présente, des fibres de la couche (2) de non-tissé étant présentes dans les régions de liaison (5), orientées et/ou étirées, la couche élastique (1) sous forme de film élastique présentant un poids spécifique par unité de surface de 10 à 130 g/m².

2. Elément de couche selon la revendication 1, **caractérisé en ce que** les régions de liaison (5) présentent des zones extérieures (9) dans lesquelles aucun matériau de la couche élastique (1) n'a pénétré, de sorte qu'aucun matériau de la couche élastique (1)
n'a pénétré la couche de non-tissé (2).

3. Elément de couche selon la revendication 2, **caractérisé en ce que** dans les zones extérieures (9), le matériau non tissé n' pas fondu.

4. Elément de couche selon l'une des revendications 1 à 3, **caractérisé en ce que**, dans les zones (8) dans lesquelles une liaison par complémentarité de forme entre le matériau non tissé et le matériau élastique solidifié est réalisée, le matériau non tissé n'est pas fondu.

5. Elément de couche selon l'une des revendications 1 à 4, **caractérisé en ce que** la couche (2) de non-tissé est constituée d'un non-tissé consolidé par jet d'eau, de préférence d'un non-tissé de filaments continus consolidé par jet d'eau, ledit non-tissé présente de préférence un poids spécifique de 10 à 70 g/m², en particulier de 10 à 40 g/m², de préférence de 15 à 35 g/m².

6. Elément de couche selon l'une des revendications 1 à 5, **caractérisé en ce que** la couche élastique (1) est constituée de plusieurs couches et présente de préférence une couche centrale (3) et au moins une autre couche (4).

7. Elément de couche selon l'une des revendications 1 à 6, **caractérisé en ce que** l'élément comprend une autre couche de non-tissé, la couche élastique (1) étant disposée entre les deux couches de non-tissé.
